# EUROPÄISCHE PATENTSCHRIFT

(11) **EP 2 989 446 B1**
(45) Veröffentlichungstag und Bekanntmachung des Hinweises auf die Patenterteilung: **03.10.2018**
(21) Anmeldenummer: 14716274.7
(22) Anmeldetag: 04.04.2014
(51) Int. Cl.: G01N 21/59, G01N 21/17

(54) **VERFAHREN ZUR AUTOMATISIERTEN AUSWERTUNG VON INKUBIERTEN IMMUNBLOTSTREIFEN**
METHOD OF AUTOMATED EVALUATION OF INCUBATED IMMUNOBLOT STRIPS
PROCÉDÉ D'ÉVALUATION AUTOMATIQUE DES BANDELETTES DES IMMUNOBLOTS INCUBEÉS

(30) Priorität: 24.04.2013 DE 102013007045; 21.05.2013 DE 102013008468
(43) Veröffentlichungstag der Anmeldung: 02.03.2016
(73) Patentinhaber: Euroimmun Medizinische Labordiagnostika AG, 23560 Lübeck (DE)
(72) Erfinder: MEYER, Wolfgang, 23689 Pansdorf (DE); SCHEPER, Thomas, 23919 Berkenthin (DE); KAFFKA, Robert, 23942 Dassow (DE)
(86) Internationale Anmeldenummer: PCT/EP2014/056787
(87) Internationale Veröffentlichungsnummer: WO 2014/173657

(56) Entgegenhaltungen:
- WO-A2-99/53288
- DE-A1- 10 109 777
- DE-T2- 3 789 381
- DE-T2- 60 213 986
- DE-T2- 68 915 267
- DE-T2- 69 933 986
- CHEN YUZHI ET AL: "Amyloid precursor protein modulates beta-catenin degradation", JOURNAL OF NEUROINFLAMMATION, BIOMED CENTRAL LTD., LONDON, GB, Bd. 4, Nr. 1, 10. Dezember 2007 (2007-12-10), Seite 29, XP021037916, ISSN: 1742-2094
- KWON H ET AL: "Caveolin-2 regulation of STAT3 transcriptional activation in response to insulin", BIOCHIMICA ET BIOPHYSICA ACTA. MOLECULAR CELL RESEARCH, ELSEVIER SCIENCE PUBLISHERS, AMSTERDAM, NL, Bd. 1793, Nr. 7, 7. Mai 2009 (2009-05-07), Seiten 1325-1333, XP026684229, ISSN: 0167-4889, DOI: 10.1016/J.BBAMCR.2009.04.015 [gefunden am 2009-05-07]
- HOLMSETH S ET AL: "The concentrations and distributions of three C-terminal variants of the GLT1 (EAAT2; slc1a2) glutamate transporter protein in rat brain tissue suggest differential regulation", NEUROSCIENCE; [1068-7971], NEW YORK, NY, US, Bd. 162, Nr. 4, 15. September 2009 (2009-09-15), Seiten 1055-1071, XP026436784, ISSN: 0306-4522 [gefunden am 2009-03-27]
- SLADEK C D ET AL: "Characterization of nuclear neurokinin 3 receptor expression in rat brain", NEUROSCIENCE; [1068-7971], NEW YORK, NY, US, Bd. 196, 19. August 2011 (2011-08-19), Seiten 35-48, XP028316200, ISSN: 0306-4522, DOI: 10.1016/J.NEUROSCIENCE.2011.08.044 [gefunden am 2011-09-08]
- Ahmed Elbaggari ET AL: "Imaging of Chemiluminescent Western Blots: Comparison of Digital Imaging and X-ray Film", Bio-Rad Tech Note 5809, 1. Januar 2008 (2008-01-01), XP055125751, Gefunden im Internet: URL:http://www.bio-rad.com/webroot/web/pdf /lsr/literature/Bulletin_5809.pdf [gefunden am 2014-06-27]

## Beschreibung

Die Erfindung betrifft ein Verfahren zur automatisierten Auswertung von inkubierten Lineblotstreifen. Wesentlich für derartige Lineblotstreifen ist, dass diese mit Antigenen beschichtete Bereiche aufweisen, die sich unter Bildung so genannter Banden verfärben sobald eine Bindung insbesondere zwischen dem Antigen und einem in einer Patientenprobe vorhandenen Antikörper auftritt.
Im Bereich der In-vitro-Diagnostik werden zur Diagnoseerstellung vielfach Immunblotstreifen, wie etwa Western-Blot-, oder Line-Blot-Streifen, eingesetzt. Die jeweils benötigten Immunblotstreifen werden in hierfür vorgesehene Inkubationsrinnen eingelegt, in denen sie mit den erforderlichen Reagenzien sowie der Patientenprobe inkubiert werden. Nach einer korrekten Testdurchführung erscheinen bei positiv getesteten Patientenproben auf den Immunblotstreifen spezielle Verfärbungen, die so genannten Banden, wodurch insbesondere die Bindung von Antikörpern an die auf den Immunblotstreifen vorgesehenen Antigene nachweisbar ist. Die Auswertung der inkubierten Immunblotstreifen kann entweder durch eine Sichtprüfung erfolgen oder aber die Immunblotstreifen werden mit Hilfe automatisierter oder zumindest teilautomatisierter Systeme ausgewertet. In diesem Zusammenhang ist die sogenannte EU-ROBlotCamera von der Firma EUROIMMUN Medizinische Labordiagnostika AG bekannt, die Aufnahmen von inkubierten Immunblotstreifen macht, während sich die Streifen noch in den Inkubationsrinnen einer Inkubationswanne befinden. Die entsprechenden Aufnahmen werden digitalisiert, an eine Datenauswerteeinheit übertragen und dort mit Hilfe einer speziellen Laborsoftware ausgewertet. Bei der Auswertung der Immunblotstreifen wird zur Vorbereitung der Erstellung eines Diagnosevorschlags zunächst das Auftreten der Banden sowie die Verfärbung der einzelnen Banden im Vergleich zum Hintergrund ermittelt. Bei stark positiven Proben kommt es regelmäßig zu einer starken Verfärbung auf den Immunblotstreifen, während lediglich schwach positive Proben oftmals nur schwache und somit schwer auszuwertende Signale liefern.

Ein ähnliches System ist aus der WO99/53288 bekannt, in der ein automatisiertes System zur Auswertung von Borrelien-Blots beschrieben wird. Auch hier werden mit Hilfe einer Digitalkamera Aufnahmen der inkubierten Immunblotstreifen gemacht und an eine elektronische Datenverarbeitungseinheit zur Auswertung übertragen. Die Verfärbung der Banden bei positiven Patientenproben wird wiederum nach Farbstufen aufgelöst ausgewertet. Problematisch bei der Verwendung der beschriebenen Systeme ist oftmals, dass unterschiedliche Membrane als Substrat für die Herstellung für Immunblotstreifen verwendet werden. Insbesondere kommen Membrane auf Basis von PVDF, Nylon oder Nitrozellulose zum Einsatz. In Bezug auf die Erstellung von Aufnahmen der inkubierten Immunblotstreifen ist hierbei zu beachten, dass sich das Verfärbungsverhalten der Immunblotstreifen aus unterschiedlichen Membranen oftmals deutlich unterscheidet, was für eine automatisierte Auswertung der Verfärbungen problematisch sein kann. Weiterhin ist das Trocknungsverhalten der zuvor erwähnten Materialien unterschiedlich, so dass teilweise Aufnahmen von Immunblotstreifen gemacht werden, die eine unterschiedliche Restfeuchte aufweisen. Die Restfeuchte wiederum hat direkten Einfluss auf die Hintergrundfarbe eines Immunblotstreifens und somit auf die Ermittlung des Farbunterschiedes zwischen Bande und Hintergrund des Immunblotstreifens. Weiterhin ist zu beachten, dass selbst Einzelaufnahmen von Immunblotstreifen mit fester Belichtungszeit, mit definierter Ausleuchtung und/oder nach festgelegter Trocknungszeit nicht immer ein optimales Bild hinsichtlich der Hintergrundfärbung oder des Verhältnisses zwischen Bandenfärbung und Hintergrundfärbung liefern oder es kommt zu einem Überstrahlen des Bildes, was letztendlich zu einer erschwerten Bildauswertung führt.

In CHEN YUZHI ET AL, "Amyloid precursor protein modulates beta-catenin degradation", JOURNAL OF NEUROINFLAMMATION, BIOMED CENTRAL LTD., LONDON, GB, (20071210), vol. 4, no. 1, ISSN 1742-2094, page 29, wird ein sogenanntes Westernblotverfahren beschrieben, bei welchem Chemielumineszenzreaktionen mittels digitaler Bildaufnahmen erfasst werden. Hierbei werden mehrere Aufnahmen eines solchen Westernblots erfasst dann eine einzelne Aufnahme verwendet, um eine Densitometrie bzw. Dichtemessung durchzuführen. Es wird ferner beschrieben, das Westernblotexperiment drei Mal oder auch häufiger durchzuführen.

In KWON H ET AL, "Caveolin-2 regulation of STAT3 transcriptional activation in response to insulin", BIOCHIMICA ET BIOPHYSICA ACTA. MOLECULAR CELL RESEARCH, ELSEVIER SCIENCE PUBLISHERS, AMSTERDAM, NL, vol. 1793, no. 7, doi:10.1016/J.BBAMCR.2009.04.015, ISSN 0167-4889, (20090507), pages 1325 - 1333, wird vorgeschlagen, bei einem Immunblot-Experiment mit Chemielumineszenzfärbung mehrere Aufnahmen eines Blots zu erfassen und für die einzelnen Banden die jeweiligen Bilder darzustellen.

In HOLMSETH S ET AL, "The concentrations and distributions of three C-terminal variants of the GLT1 (EAAT2; slc1a2) glutamate transporter protein in rat brain tissue suggest differential regulation", NEUROSCIENCE; [1068-7971], NEW YORK, NY, US, vol. 162, no. 4, ISSN 0306-4522, (20090915), pages 1055 - 1071, wird vorgeschlagen, bei einem Blottingverfahren mit Chemilumineszenzfärbung Bilddaten zu erfassen und unterschiedliche Aufnahmen eines gleichen Westernblots zu gewinnen.

In SLADEK C D ET AL, "Characterization of nuclear neurokinin 3 receptor expression in rat brain", NEUROSCIENCE; [1068-7971], NEW YORK, NY, US, vol. 196, doi:10.1016/J.NEUROSCIENCE.2011.08.044, ISSN 0306-4522, (20110819), pages 35 - 48, wird vorgeschlagen, bei einem Westernblot mehrere Aufnahmen des Blots zu erfassen und eine Analyse hinsichtlich der optischen Dichte mittels einer Software durchzuführen.

In Ahmed Elbaggari ET AL, "Imaging of Chemiluminescent Western Blots: Comparison of Digital Imaging and X-ray Film", Bio-Rad Tech Note 5809, (20080101), URL: http://www.bio-rad.com/webroot/web/pdf/Isr/Literature/Bulletin_5809.pdf, (20140627), wird vorgeschlagen, bei Westernblotverfahren, bei welchen eine Chemielumineszenzverfärbung festgestellt werden kann, mittels eines Filmmaterials Ablichtungen des Blotergebnisses zu gewinnen gewonnen werden und diese Filme nach ihrer Entwicklung dann mittels eines bildgebenden Verfahrens bzw. einer Bildgebungsvorrichtung hinsichtlich der optischen Dichte zu analysieren. Hierbei wird ferner vorgeschlagen, für ein Blotergebnis, welches schwache als auch starke Signale aufweist, mehrere Aufnahmen zu erfassen.

Ausgehend von den bekannten Systemen zur automatisierten Auswertung von Immunblotstreifen sowie den zuvor geschilderten Problemen liegt der Erfindung die Aufgabe zu Grunde, ein System anzubieten, mit dem eine qualitativ hochwertige Auswertung von Lineblotstreifen ermöglicht wird. Es soll die erfindungsgemäße technische Lösung sicherstellen, dass insbesondere das Verhältnis zwischen Verfärbung der einzelnen Banden und der Hintergrundfärbung eine sichere Auswertung zulässt und die Gefahr des so genannten Überstrahlens weitgehend ausgeschlossen wird. Das verbesserte Verfahren soll sich ferner auf einfache Weise in den bekannten Laborablauf integrieren und sich mit verhältnismäßig einfachen Mitteln realisieren lassen.

Die zuvor geschilderte Aufgabe wird mit Hilfe eines Verfahrens gemäß Anspruch 1 realisiert. Vorteilhafte Ausführungsformen der Erfindung sind Gegenstand der abhängigen

Ansprüche und werden in der folgenden Beschreibung unter teilweiser Bezugnahme auf die Figuren näher erläutert.

Erfindungsgemäß ist ein Verfahren zur automatisierten Auswertung eines inkubierten Lineblotstreifen, bei dem mit einer Kamera zumindest bereichsweise von einer Oberfläche eines Lineblotstreifens mit einzelnen darauf angebrachten Teststücken , auf der Antigene angeordnet sind, insbesondere von den so genannten verfärbten Banden, Bilder aufgenommen werden und die Bilder an eine Auswerteeinheit übertragen werden, in der eine Verfärbung des Lineblotstreifens in Bereichen der aufgebrachten Antigene detektiert und auf der Grundlage der detektierten Verfärbung ein Diagnosevorschlag generiert wird, derart weitergebildet worden, dass mit der Kamera jeweils wenigstens zwei Aufnahmen zu unterschiedlichen Zeitpunkten mit unterschiedlichen Belichtungszeiten gemacht werden und dass für die einzelnen Teststücke die jeweils optimalen Aufnahmen herausgefiltert werden und als ein optimiertes Gesamtbild eines inkubierten Lineblots der Generierung des Diangosevorschlags zugrundegelegt werden.
In einer bevorzugten Ausführungsform umfassen die wenigstens zwei Aufnahmen zu unterschiedlichen Zeitpunkten eine erste und eine letzte Aufnahme, optional zusätzliche Aufnahmen dazwischen, wobei der zeitliche Abstand zwischen der ersten und der letzten Aufnahme wenigstens 0,5, 1, 2, 3, 4, 5, 7,5, 10, 12,5, 15, 20, 25, 30, 35, 40, 45, 50, 60, 80, 90, 100, 120, 150 oder 180 Minuten, noch bevorzugter wenigstens 5, 10 oder 15, am bevorzugtesten wenigstens 5 Minuten beträgt.

Wesentlich an dem erfindungsgemäßen Verfahren ist somit, dass die Bedingungen, unter denen die einzelnen Aufnahmen des Lineblotstreifens gemacht werden, variieren und zur Generierung eines Diagnosevorschlags letztendlich die am besten geeigneten Aufnahmen verwendet werden. Im Rahmen der Auswertung wird somit quasi bedarfsgerecht ein virtueller Immunblotstreifen aus den geeignetsten Bildern der verschiedenen Aufnahmen zusammengesetzt. Die für die Generierung eines Diagnosevorschlags verwendeten Bilder der verfärbten Banden können somit von einer oder von verschiedenen Aufnahmen stammen. Die Veränderung der Aufnahmebedingungen kann hierbei darin bestehen, dass verschiedenen Aufnahmen zu unterschiedlichen Zeiten, insbesondere zu unterschiedlichen Trocknungszeiten, gemacht werden.

Erfindungsgemäß ist es vorgesehen, dass wenigstens zwei Aufnahmen von der mit Antigenen beschichteten Oberfläche eines Lineblotstreifen gemacht werden, wobei sich die Belichtungszeiten der unterschiedlichen Aufnahmen unterscheiden. Besonders bevorzugt werden wenigstens zwei Aufnahmen von jeweils wenigstens zwei Lineblotstreifen gemacht.

Die unterschiedlichen Aufnahmen werden in jedem Fall an die Auswerteeinheit übertragen, in der die Verfärbung der einzelnen Bereiche, die so genannten Banden, mit der Hintergrundfärbung des jeweils dazugehörigen Lineblotstreifens verglichen und mit Hilfe einer Farbstufenskala, die die ganzzahligen Werte 0 bis 255 aufweist, klassifiziert wird. Zunächst werden hierbei die unterschiedlichen Aufnahmen miteinander verglichen und die jeweiligen Aufnahmen eines Lineblotstreifens für die Generierung eines Diagnosevorschlag verwendet, deren Farbunterschied zwischen den einzelnen Banden und dem Streifenhintergrund besonders groß ist. Besonders bevorzugt werden helle Aufnahmen verwendet.
In der Auswerteeinheit werden somit aus den unterschiedlichen Aufnahmen die optimalen herausgefiltert um diese für die Generierung des Diagnosevorschlags zu verwenden. In diesem Zusammenhang ist es denkbar, dass für Lineblots oder Membranchips mit einzelnen darauf angeordneten Teststücken, für jedes Teststück die optimale Aufnahme der Verfärbung ermittelt wird. Stets werden somit zunächst die für die Auswertung am besten geeigneten Banden eines Streifens herausgefiltert und diese Banden für die automatisierte Erstellung eines Diagnosevorschlags berücksichtigt. In diesem Zusammenhang ist vorgesehen, dass die einzelnen Aufnahmen der Banden eines Lineblotstreifens verglichen werden und die jeweils beste Aufnahme einer Bande zur Generierung eines Diagnosevorschlags verwendet wird. Gemäß dieser Ausführungsform ist es denkbar, dass während der Erstellung eines Diagnosevorschlags ein quasi virtueller Immunblotstreifen berücksichtigt wird, der sich aus Aufnahmen von Banden zusammensetzt, die zu unterschiedlichen Zeitpunkten und mit unterschiedlicher Belichtungszeit aufgenommen worden sind. Mit Hilfe dieser Maßnahme ist es somit möglich, eine Vielzahl von Aufnahmen zu unterschiedlichen Zeitpunkten und die jeweils besten Aufnahmen für die Erstellung eines Diagnosevorschlags zu verwenden. Die Qualität bei der Erstellung eines Diagnosevorschlags wird somit deutlich gesteigert.

In Ergänzung zur Variation der Belichtungszeit wenigstens zweier Aufnahmen ist es ebenfalls denkbar verschiedene Aufnahmen bei unterschiedlicher Helligkeit bzw. Beleuchtung des Immunblotstreifens zu erstellen. Wesentlicher Gedanke ist auch hierbei, mehrere Aufnahmen zu generieren und letztendlich die für die Auswertung am besten geeignetsten zu verwenden, wobei ggf. auch unterschiedliche Banden verschiedener Aufnahmen berücksichtigt werden.
In einer bevorzugten Ausführungsform unterscheidet sich die Helligkeit, bevorzugt ausgedrückt als Beleuchtungsstärke, zwischen der Aufnahme mit der höchsten Helligkeit und der Aufnahme mit der niedrigsten Helligkeit unter den wenigstens zwei Aufnahmen um wenigstens 5%, 10%, 15%, 20%, 25%, 30%, 40%, 50%, 60%, 70%, 80%, 90%, 100%, 125%, 150%, 175% oder 200% der Helligkeit der Aufnahme mit der niedrigsten Helligkeit.

Gemäß einer speziellen Weiterbildung werden wenigstens zwei Aufnahmen bei Belichtungszeiten zwischen 20 und 50 Millisekunden aufgenommen.

Ausdrücklich wird in diesem Zusammenhang erwähnt, dass sich das erfindungsgemäße Verfahren besonders gut für die Auswertung von Lineblot-Streifen eignet.

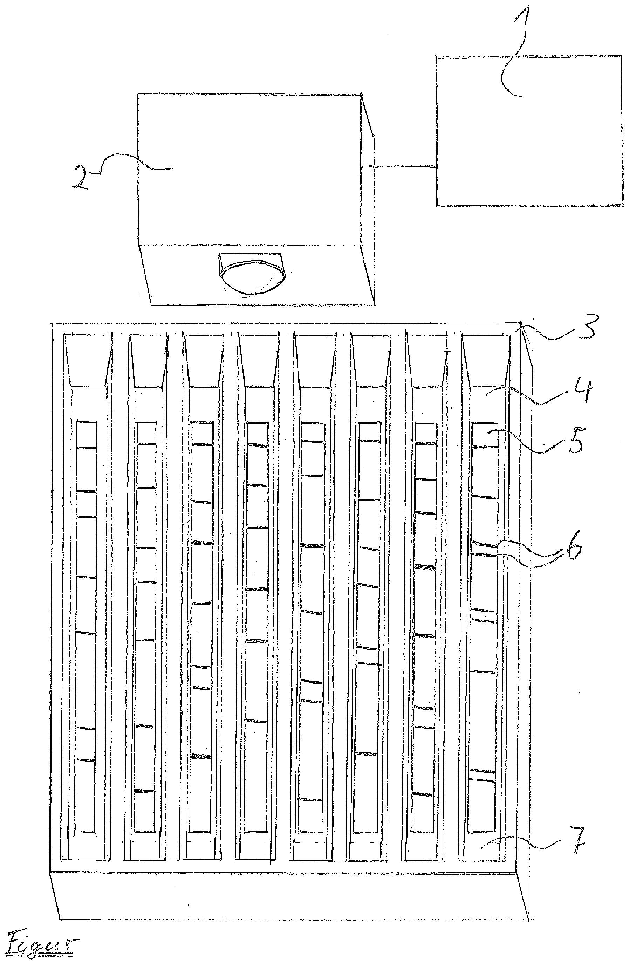

Im Folgenden wird die Erfindung ohne Beschränkung des allgemeinen Erfindungsgedankens anhand eines Ausführungsbeispiels unter Zuhilfenahme der Figur näher erläutert. Dabei zeigt:
- Figur:: Inkubationswanne mit einer Vielzahl von Inkubationsrinnen, in denen sich inkubierte Lineblotstreifen befinden, die mit Hilfe einer Digitalkamera aufgenommen werden.

Die Figur zeigt eine oberhalb einer Inkubationswanne 3, die eine Vielzahl von Inkubationsrinnen 4 aufweist, angeordnete Kamera 2, die Aufnahmen von in den Rinnen 4 liegenden Lineblotstreifen 5 macht und in digitalisierter Form an eine Auswerteeinheit 1 überträgt. Die in den einzelnen Rinnen 4 angeordneten Lineblotstreifen 5 wurden bereits inkubiert und sind zumindest teilweise bereits getrocknet. Deutlich zu erkennen ist, dass die verschiedenen Lineblotstreifen 5 an unterschiedlichen Positionen Verfärbungen in Form von Banden 6 aufweisen. An diesen Stellen befinden sich auf den Oberflächen der Teststreifen 5 Antigene, die mit Antikörpern einer untersuchten Patientenprobe eine positive Reaktion zeigen. Auf diese Weise kann ein Diagnosevorschlag generiert werden, der eine Auskunft darüber gibt, wie hoch die Wahrscheinlichkeit ist, dass ein Patient an einer bestimmten Krankheit erkrankt ist. Ausdrücklich wird darauf hingewiesen, dass es für das durchgeführte Verfahren unerheblich ist, ob nur eine Inkubationsrinne mit einem Blotstreifen gefüllt ist oder ob sich in wenigstens zwei Rinnen Blotstreifen befinden. Üblicherweise werden bereits aufgrund arbeitsökonomischer und wirtschaftlicher Gesichtspunkte mehrere Rinnen einer Inkubationswanne mit Lineblotstreifen bestückt und diese gleichzeitig inkubiert und anschließend ausgewertet.

Die Auswertung der inkubierten Lineblotstreifen 5 erfolgt automatisiert mit Hilfe einer Digitalkamera 2. Die Digitalkamera 2 macht hintereinander insgesamt fünf Aufnahmen von den Oberflächen der in den Rinnen 4 befindlichen Lineblotstreifen 5, die sich in Bezug auf die Belichtungszeit unterscheiden. Es werden Aufnahmen mit einer Belichtungszeit von 21, 28, 35, 42 und 49 ms erstellt. Von jedem inkubierten, in einer Inkubationsrinne 4 befindlichen Lineblotstreifen 5 werden somit schließlich fünf digitalisierte Aufnahmen an die Auswerteeinheit 1 übermittelt. In der Auswerteeinheit 1 wird für die verschiedenen Aufnahmen zunächst die Verfärbung der einzelnen Banden im Vergleich zur Verfärbung des Hintergrunds 7 ermittelt und jeder dieser Bandenverfärbungen ein die jeweilige Farbstufe repräsentierender Zahlenwert, der ganzzahlig ist und zwischen 0 und 255 liegt, zugeordnet.

In einem nächsten Schritt werden die Banden 6 der einzelnen Lineblotstreifen 5 ausgewählt, die die deutlichste Verfärbung im Vergleich zum Hintergrund 7 zeigen. In Bezug auf einen Lineblotstreifen 5 werden schließlich die besten Aufnahmen, nämlich die den deutlichsten Farbunterschied einer Bande 6 gegenüber dem jeweiligen Hintergrund 7 aufweisen, ausgesucht, wobei die verschiedenen ausgewählten Aufnahmen zu unterschiedlichen Zeitpunkten und in diesem Fall mit unterschiedlichen Belichtungszeiten aufgenommen sind. Die als optimale Aufnahmen herausgefilterten Aufnahmen der Bandenverfärbungen werden schließlich mit Hilfe einer elektronischen Laborsoftware ausgewertet und so ein Diagnosevorschlag generiert. Durch das beschriebene Verfahren ist es möglich, bei der Verwendung von Linienblots mit einzelnen aufgebrachten, antigentragenden Membranchips, die jeweils optimalen Aufnahmen der einzelnen Membranchips herauszufiltern und als quasi optimiertes Gesamtbild eines inkubierten Immunblotstreifens der Generierung eines Diagnosevorschlags zugrunde zu legen.

### Bezugszeichenliste

- 1: Auswerteeinheit
- 2: Kamera
- 3: Inkubationswanne
- 4: Inkubationsrinne
- 5: inkubierter Immunblotstreifen
- 6: Bande
- 7: Hintergrund

## Patentansprüche

1. Verfahren zur automatisierten Auswertung eines inkubierten Lineblots mit einzelnen darauf angebrachten Teststücken, bei dem mit einer Kamera (2) zumindest bereichsweise von einer Oberfläche des Lineblots, auf der Antigene angeordnet sind, Bilder aufgenommen werden und die Bilder an eine Auswerteeinheit (1) übertragen werden, in der eine Verfärbung des Lineblots in Bereichen der aufgebrachten Antigene detektiert und auf der Grundlage der detektierten Verfärbung ein Diagnosevorschlag generiert wird,
**dadurch gekennzeichnet, dass** mit der Kamera (2) jeweils wenigstens zwei Aufnahmen zu unterschiedlichen Zeitpunkten und mit unterschiedlichen Belichtungszeiten gemacht werden, und dass für die einzelnen Teststücke die jeweils optimalen Aufnahmen herausgefiltert werden und als ein optimiertes Gesamtbild eines inkubierten Lineblots der Generierung des Diagnosevorschlags zugrundegelegt werden.

2. Verfahren nach einem der Anspruch 1,
**dadurch gekennzeichnet, dass** fünf Aufnahmen mit unterschiedlichen Belichtungszeiten aufgenommen werden.

3. Verfahren nach einem der Ansprüche 1 oder 2 ,
**dadurch gekennzeichnet, dass** die wenigstens zwei Aufnahmen bei Belichtungszeiten zwischen 20 und 50 ms aufgenommen werden.

4. Verfahren nach einem der Ansprüche 1 bis 3,
**dadurch gekennzeichnet, dass** ein Farbunterschied der Bereiche mit aufgebrachten Antigenen gegenüber dem Hintergrund (7) ermittelt wird.

5. Verfahren nach Anspruch 4,
**dadurch gekennzeichnet, dass** der Farbunterschied in ganzzahligen Stufen zwischen 0 und 255 einklassifiziert wird.

## Claims

1. Method for automated analysis of an incubated line blot with single affixed test parts, whereby pictures of at least areas of a surface of the line blot, on which antigens are arranged, are taken with a camera (2) and the pictures are transferred to an analysis unit (1), in which a discolouration of the line blot in areas of attached antigens is detected and a diagnostic suggestion is generated based on the detected discolouration,
**characterized in that** at least two images are taken by the camera (2) at different time instances and with different exposure times, respectively, and **in that** the respective optimal images of the single test parts are filtered out and are taken into account as a combined overall picture of an incubated line blot as a basis for the generation of the diagnostic suggestion.

2. Method according to claim 1, **characterized in that** five records are taken with different exposure times.

3. Method according to any one of claims 1 or 2, **characterized in that** the at least two images are taken with exposure times between 20 and 50 ms.

4. Method according to any one of claims 1 to 3, **characterized in that** a colour difference of the areas with arranged antigens is determined with respect to the background (7).

5. Method according to claim 4, **characterized in that** the colour difference is classified into integer steps between 0 and 255.

## Revendications

1. Procédé d'interprétation automatique d'un lineblot incubé comprenant des éprouvettes individuelles disposées sur celui-ci, selon lequel des images au moins de certaines zones d'une surface du lineblot sur laquelle sont appliqués des antigènes sont enregistrées par une caméra (2) et les images sont transmises à une unité d'interprétation (1) dans laquelle une coloration du lineblot dans les zones des antigènes appliqués est détectée et une proposition de diagnostic est générée sur la base de la coloration détectée,
**caractérisé en ce qu'**au moins deux enregistrements sont respectivement effectués avec la caméra (2) à des instants différents et avec des temps d'exposition différents, et **en ce que** les enregistrements respectivement optimaux sont extraits par filtrage pour les éprouvettes individuelles et servent de base à la génération de la proposition de diagnostic sous la forme d'une image globale optimisée d'un lineblot incubé.

2. Procédé selon la revendication 1, **caractérisé en ce que** cinq enregistrements sont enregistrés avec des temps d'exposition différents.

3. Procédé selon l'une des revendications 1 ou 2, **caractérisé en ce que** les au moins deux enregistrements sont enregistrés avec des temps d'exposition entre 20 et 50 ms.

4. Procédé selon l'une des revendications 1 à 3, **caractérisé en ce qu'**une différence de couleur par rapport à l'arrière-plan (7) des zones où les antigènes sont appliqués est déterminée.

5. Procédé selon la revendication 4, **caractérisé en ce que** la différence de couleur est classifiée en niveaux de nombres entiers comrpis entre 0 et 255.
